# EUROPEAN PATENT APPLICATION

(11) **EP 4 628 598 A1**
(43) Date of publication of application: **08.10.2025**
(21) Application number: 24168375.4
(22) Date of filing: 04.04.2024
(51) Int. Cl.: C12Q 1/6886

(54) **DNA METHYLATION MARKER FOR DETERMINATION OF T-STATUS OF CANCER**

(71) Applicant: Siemens Healthineers AG, 91301 Forchheim (DE)
(72) Inventor: Fellendorf, Sandra, 50827 Köln (DE); Klatt, Torbjörn, 50374 Erftstadt (DE); Leenders, Frauke, 42699 Solingen (DE)
(74) Representative: Siemens Healthineers Patent Attorneys

(57) **Abstract**

The present invention relates to a method for diagnosing, detecting or monitoring a cancer disease in a subject, comprising determining the methylation level of a methylation site in a genomic DNA derived from the subject's sample, wherein said methylation site comprises the differentially methylated position (DMP) corresponding to Illumina probe ID cg01218619 (SEQ ID NO: 1). Also envisaged is a corresponding kit, a method of enriching DNA molecules derived from a subject's sample and the use of the DMP for diagnosing, detecting or monitoring a cancer disease.

## Description

### TECHNICAL FIELD

The present invention relates to a method for diagnosing, detecting or monitoring a cancer disease in a subject, comprising determining the methylation level of a methylation site in a genomic DNA molecule derived from the subject's sample, wherein said methylation site comprises the differentially methylated position (DMP) corresponding to Illumina probe ID cg01218619 (SEQ ID NO: 1) and wherein the diagnosing, detecting or monitoring of a cancer disease in a subject comprises a distinction between a TNM-classification T1 status and T3/T4 status in the subject. Also envisaged is a corresponding kit, a method of enriching DNA molecules derived from a subject's sample and the use of the DMP for diagnosing, detecting or monitoring a cancer disease.

### BACKGROUND

Lung cancer is the most common cancer and the leading cause of cancer-related morbidity worldwide with about 2 million new cases per year. Typical determinants of cancers, independent of the specific entities, are the site of the tumor, the tumor profile (e.g., histopathology, morphology, molecular and genetic characteristics etc.) and tumor stage.

Staging refers to the classification of cancer by disease extent and is a major determinant of appropriate treatment selection and prognosis. One classification method, called TNM-staging, is a unified standard for the staging of tumors. This multidimensional classification indicates different degrees of severity regarding the extent (size, depth of infiltration) of the primary tumor (T), lymph node involvement (N) and presence of distant metastases (M). The T-descriptor, also referred to as T-status, describes extent of size and depth of infiltration of the primary tumor. An extensive evaluation of the TNM tumor T-descriptor therefore requires a multimodal approach including imaging to evaluate the tumor size, and invasive histopathology and morphology assessment to evaluate tumor infiltration.

Oncological diagnostics is a multi-step process based on a combination of non- or semi-invasive testing, including diagnostic bronchoscopy and bronchoalveolar lavage, imaging, and invasive histopathological evaluation. Newer approaches, which are not yet the clinical standard, are also attempting to detect biochemical and genetic material of the tumor from blood using minimal-invasive liquid biopsies, possibly enabling earlier detection and faster therapy induction.

Since, under ideal conditions, therapy can only begin after the establishment of pathological diagnosis and proper staging, this becomes a rate limiting step for all downstream processes. If a pathological diagnosis is not possible for whatever reasons, therapy may still be carried out using, for example, imaging and optionally laboratory tests. Overall access to care and hospital processes are the two most common reasons for delays in cancer diagnosis and treatment initiation. A study of Verma and colleagues (Verma et al., 2015, Medicine, 94(29), e1216) revealed an inappropriate long time to diagnosis in up to 40 % of patients.

The coronavirus pandemic has exposed the limitations of global healthcare systems. These limitations were for example evident, as U.S. hospitals had to suspend their cancer care practices and functions at the onset of the 2020 pandemic in order to allocate resources to treat the highly increasing number of patients with COVID-19 (e.g., Hanna et al., 2020, BMJ, 371, m4087) .

Routine procedures, including cancer screening, diagnosis, and treatment (e.g., surgery, chemotherapy, radiotherapy), have been postponed or delayed accommodating the situation. This situation was and is exacerbated by labor shortage and associated inaccessibility or significantly extended waiting times to a significant loss in access to medical care (e.g., Woolhandler et al., 2020; Ann Intern Med., 173(1) :63-64).

Staff shortage, low availability in rural regions or too high requests in metropolitan areas may additionally lead to a shortage of resources and have a significant negative impact on cancer patient outcome, as already delays of a few weeks may significantly increase the risk of metastases in some fast-growing tumor entities (Ng et al., 2021, JAMA Netw Open, 4(1): e2034065) .

Additionally, suboptimal biopsy quality or inability to perform proper biopsy, due to proximity to major organs, blood vessel or nerve pathways, or unfit patients, lead to missing histopathological information, which in turn may negatively impact TNM-staging and therapy selection based on the associated omission of histopathological analysis.

Therefore, the above-mentioned constraints of the healthcare system might lead to a delayed diagnosis of cancer patients, in particular lung cancer patients.

There is hence a need for novel and effective non-invasive methods, which are independent from imaging and allow for early diagnosis or prioritization of high stage cancer patients.

### SUMMARY

The present invention addresses these needs and provides in a first aspect a method for diagnosing, detecting or monitoring a cancer disease in a subject, comprising determining the methylation level of a methylation site in a genomic DNA molecule derived from the subject's sample, wherein said methylation site comprises the differentially methylated position (DMP) corresponding to Illumina probe ID cg01218619 (SEQ ID NO: 1) and wherein the diagnosing, detecting or monitoring of a cancer disease in a subject comprises a distinction between a TNM-classification T1 status and T3/T4 status in the subject. The currently claimed method provides several advantages in comparison to approaches of the prior art. To start with, the herein described CpG-site is capable of diagnosing, detecting or monitoring a cancer disease by allowing to discriminate between a TNM-classification T1 status and T3/T4 status of a cancer disease such as lung cancer. Due to the shortage of resources and increasing number of patients, proper prioritization of patients into clinical pathways is a crucial process of modern medicine. The establishment of the currently claimed marker advantageously facilitates the prioritization of patients into clinical pathways prior to histopathological examination. This offers the possibility to select patients in case of resource and staff shortage and to prioritize the higher T-status to rapid diagnosis and therapy selection. Further, the use of methylation profiles in the context of the currently claimed marker for tumor staging could be a measure for indication of potential aggressiveness with the help of simple sample taking, e.g. a single blood draw. This significantly facilitates the diagnostic process for the individual patient and allows for discrimination of patients requiring rapid diagnosis. Further, the sample taking of the method can be performed by a general practitioner, which reduces the burden on hospitals.

In a preferred embodiment, said diagnosing, detecting or monitoring of a cancer disease in a subject comprises performing a statistical prediction. Said statistical prediction preferably utilizes a machine learning approach such as logistic regression or random forest classification trained on an appropriately representative training cohort.

In a further preferred embodiment, the method additionally comprises the step of evaluation of (i) the subject's imaging-based data, ultra-low-dose CT screening, medical images and/or pathology images; and/or (ii) the subject's non-imaging-based data, such as data on age, sex, race, characteristics of cancer phenotype, histologic characteristics, stage of development of cancer, histologic subtype, detectable molecular changes, preferably on the level of the genome, transcriptome, metabolome, proteome, glycome or lipidome, biomarkers and/or laboratory tests.

In a further preferred embodiment, the results of the evaluation(s) contribute to the diagnostic conclusion with respect to the presence of a cancer disease in the subject, preferably with respect to a distinction between a TNM-classification T1 status and T3/T4 status in the subject.

In another preferred embodiment, the method comprises the step of reporting the diagnostic conclusion, i.e. the TNM-classification of the subject. In a more preferred embodiment, the method comprises the step of reporting the diagnostic conclusion in an electronic form.

In a further aspect the present invention relates to a kit comprising probes for detecting the DMP corresponding to Illumina probe ID cg01218619 (SEQ ID NO: 1).

In yet another aspect, the present invention relates to a method of enriching DNA molecules derived from a subject's sample comprising: (a) modifying the DNA molecules to allow for determination of the methylation level of methylation sites, preferably by bisulfite-conversion or enzymatic conversion; (b) contacting the modified molecules with at least one probe as defined herein above and enriching the modified DNA molecules by hybridization capture.

In a preferred embodiment, the method additionally comprises the step (c) of determining the sequence of the enriched molecule, thereby also determining the methylation level of the enriched molecule.

In a further preferred embodiment, the determined methylation level is statistically evaluated to allow for diagnosing, detecting or monitoring a cancer disease in the subject, wherein the diagnosing, detecting or monitoring of a cancer disease in a subject comprises a distinction between a TNM-classification T1 status and T3/T4 status in the subject.

In further preferred embodiments, said sample is a blood, urine, feces, saliva, or liquid biopsy sample, more preferably a blood plasma sample.

In another aspect, the present invention relates to a use of a differentially methylated position (DMP) corresponding to Illumina probe ID cg01218619 (SEQ ID NO: 1) for diagnosing, detecting or monitoring a cancer disease, wherein the diagnosing, detecting or monitoring of a cancer disease in a subject comprises a distinction between a TNM-classification T1 status and T3/T4 status in the subject.

It is particularly preferred that the cancer is lung cancer.

### BRIEF DESCRIPTION OF THE DRAWINGS

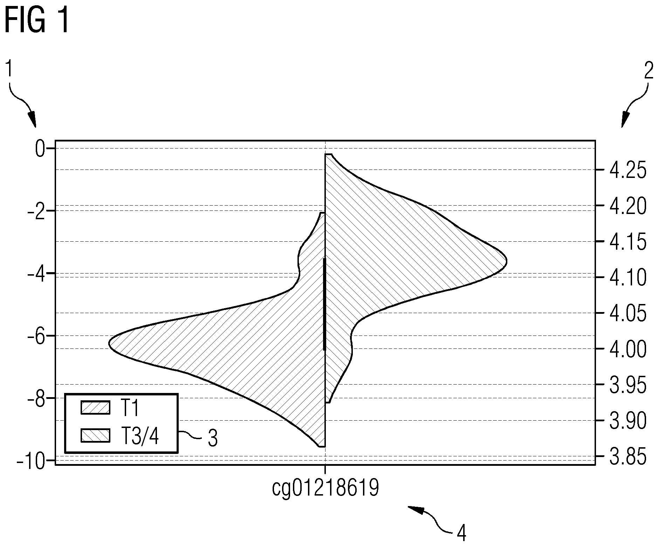

FIG. 1 shows M-values of lung cancer liquid biopsies with a tumor (T) descriptor of T1 or a combined group of T3 + T4. In particular, the figure shows the distribution of M-values of KS-significant CpG sites according to an embodiment of the invention. Indicated is the M-value **(1),** the log 10(pKS) value **(2),** and the T-Descriptor **(3)** which could either be T1 or T3/4. Further indicated is the CpG site analysed **(4).** The T1 group (hatching of type T1) shows a main distribution with lower M-values (lower methylation) compared to the T3/4 group (hatching type T3/4) with a higher distribution in M-values (higher methylation).

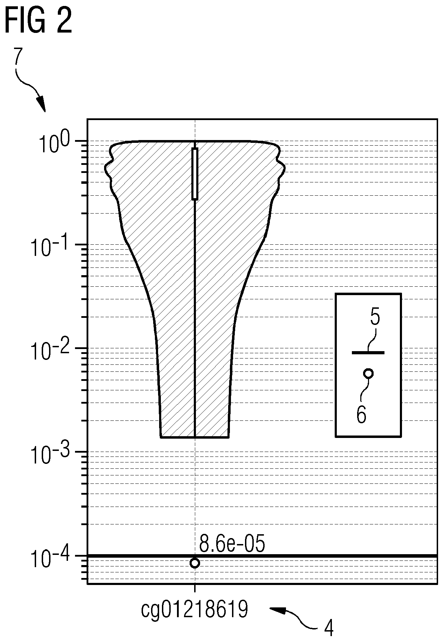

FIG. 2 depicts a distribution of p-values in a 10,000-fold randomized KS testing. Indicated are the p-value threshold **(5),** observed p-values **(6)** and, on the left hand side, the p-value **(7).** Further indicated is the CpG site analysed **(4).** In particular, the figure represents a violin plot of a multiple randomized KS testing (10,000 times the m-values of all samples at cg01218619 were distributed randomly into two groups and the respective p-values were shown at the y-axis). The observed p-value for the initial group distribution (T1 vs T3/4) is shown to be considerably different (i.e., at least an order of magnitude lower) from the multiple testing results (hatched zone) and below the p-value threshold **(5).**

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms " (i) ", "(ii)", "(iii)" or " (a) ", "(b)", "(c)", "(d)", or "first", "second", "third" etc. and the like in the description or in the claims, are used for distinguishing between similar or structural elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e., the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a method for diagnosing, detecting or monitoring a cancer disease in a subject, comprising determining the methylation level of a methylation site in a genomic DNA molecule derived from the subject's sample, wherein said methylation site comprises the differentially methylated position (DMP) corresponding to Illumina probe ID cg01218619 (SEQ ID NO: 1) and wherein the diagnosing, detecting or monitoring of a cancer disease in a subject comprises a distinction between a TNM-classification T1 status and T3/T4 status in the subject.

The term "methylation site" as used herein relates to the specific sites in a genomic DNA sequence which are modified by the addition of a methyl group to a cytosine which is converted to a 5-methylcytosine, or, in certain situations, the removal of a methyl group from the 5-methylcytosine. The methylation of DNA is a form of epigenetic modification which has the capability of altering the expression of genes and other elements and may accordingly have an effect on, e.g., silencing of tumor suppressor genes and/or increasing the expression of oncogenes, etc.

Typically, the methylation site is a 5'-cytosine-phosphate-guanine-3' (CpG) dinucleotide site. The methylation sites are distributed throughout the genome and not confined to expressed genes. In many cases, methylation sites are located in promoter or regulatory regions. The analysis of the methylation level or methylation status at the methylation site as defined herein is based on specific techniques for the detection of methyl groups. Typically, DNA molecules to be analyzed for the presence of their methylation level are specifically processed to preserve the methylation status information. Such processing may be based on techniques such as bisulfite conversion or enzymatic conversion. The "bisulfite conversion" comprises the treatment of DNA molecules with bisulfite which converts cytosine to uracil but does not convert 5-methylcytosines. Subsequent amplification, which turns uracil to thymine, is usually carried out using PCR or methylation-specific PCR (MSP). In PCR amplification uracil is recognized as thymine and the opposite strand is filled in with adenine due to complementary base pairing, while cytosines are paired with guanines in bisulfite-untreated stretches. This results in two DNA sequences (converted Cs for unmethylated and unconverted Cs for methylated), of which the converted one has C->U transitions at the unmethylated sites and is then paired with A instead of G in DNA sequencing. In the alternative method of "enzymatic conversion" instead of a chemical conversion of unmethylated cytosines an enzymatic conversion is performed. Typically, a set of two enzymes is used. For example, the enzymes TET2 and T4-BGT are used to convert 5-methylcytosines into products that cannot be deaminated by the enzyme APOBEC. In a second reaction, the enzyme APOBEC deaminates unmodified cytosines by converting them to uracils. Further analysis steps correspond to the bisulfite approach described above.

The term "cancer disease" as used herein refers to any cancer, tumor or under-controlled or uncontrolled cellular proliferation. It is preferred that the cancer disease is a lung cancer.

The term "detecting a cancer disease" as used herein means that the presence of a cancer disease or disorder in an organism may be determined or that such a disease or disorder may be identified in an organism and that it becomes possible to determine whether the subject has a TNM-classification T1 status or T3/T4 status. The determination or identification of a cancer disease or disorder according to the present invention, e.g. lung cancer, may be accomplished by a determination of the methylation level of the differentially methylated position (DMP) corresponding to cg01218619 and the comparison of the results with a suitable control or reference, e.g. a healthy or normal control or a reference previously obtained from a group of healthy or normal subjects. The method according to the present invention, based on the determination of the methylation level of cg01218619 (SEQ ID NO: 1) thus allows to determine whether a subject is affected by a cancer disease and, in particular, to distinguish between a TNM-classification T1 status and T3/T4 status in the subject.

The term "diagnosing a cancer disease" as used herein means that a subject may be considered to be suffering from a cancer disease, e.g. lung cancer, wherein said diagnosing implies the determination whether the subject has a TNM-classification T1 status or T3/T4 status, when the methylation level of the differentially methylated position (DMP) corresponding to cg01218619 is different in comparison to a control level, preferably if compared to a normal control level. The term "diagnosing" also refers to the conclusion (diagnostic conclusion) reached through that comparison process.

The term "monitoring of a cancer disease" as used herein relates to the accompaniment of a diagnosed or detected cancer disease or disorder, e.g. lung cancer, in particular the diagnosed or detected cancer with respect to the determination whether the subject has a TNM-classification T1 status or T3/T4 status. The accompaniment may be performed during a treatment procedure or during a certain period of time, typically during 2 months, 3 months, 4 months, 6 months, 1 year, 2 years, 3 years, 5 years, 10 years, or any other period of time. The term "accompaniment" means that states of disease and, in particular, changes of these sates of disease may be detected by comparing the methylation level of the differentially methylated position (DMP) corresponding to cg01218619 in a sample to a control level, preferably a control methylation level derived from a suitable control such as a T1 status subject or to the methylation level of an established, e.g. independently established, cancer cell or cell line, e.g. a lung cancer cell line or a subject having a T3/4 status, in any type of periodical time segment, e.g. every week, every 2 weeks, every month, every 2, 3, 4, 5, 6, 7, 8, 9, 10, 11 or 12 month, every 1.5 year, every 2, 3, 4, 5, 6, 7, 8,9 or 10 years, during any period of time, e.g. during 2 weeks, 3 weeks, 1, 2, 3, 4, 5, 6, 7, 8, 9, 10, 11, 12 months, 1.5, 2, 3, 4, 5, 6, 7, 8, 9, 10, 15 or 20 years, respectively. The established, e.g. independently established cancer cell, e.g. lung cancer cell, or cell line giving rise to an additional control level may be derived from samples corresponding to different stages of cancer development. In a specific embodiment of the present invention the term relates to the accompaniment of a diagnosed cancer disease, e.g. lung cancer disease. The monitoring may also include the detection of the methylation level of additional differentially methylated positions.

The methylation site to be analyzed in order to implement the present method as mentioned above can be derived from the following Table:

**Table 1**

| **No.** | **CpG-Site ID (DMP)** | **Chromosome** | **Start position** | **End position** | **Gene** | **SEQ ID NO:** |
|---|---|---|---|---|---|---|
| 1 | cg01218619 | 4 | 25088676 | 25088677 | - | 1 |

In the following Table 1a the genomic sequence surrounding the CpG sites of the DMP of Table 1 is provided in detail (with the CpG site being marked):

**Table 1a**

| **No.** | **CpG-Site ID (DMP)** | **SEQ ID NO:** | **Sequence with marked CpG site (underlined)** |
|---|---|---|---|
| 1 | cg01218619 | 1 | ggcaggggctgtgcgcggctctctcccctaaagcgag gttcgtgctcatctctagcccccgccgctctcgtggg ccacgtcc |

The DMP mentioned herein, e.g. in Tables 1 and 1a, is provided in the Illumina ID nomenclature. Information on the genomic location of the methylation sites, as well as data on associated experiments, studies and scientific references etc. can be derived, for example, from the website of Illumina (www.illumina.com). Accordingly, the identity of the DMP represented by the DMP locus identifier as depicted in Table 1 is publicly available from the Illumina website, e.g., under reference to the DMP sites used in the Infinium HumanMethylation450 BeadChip kit.

Alternatively, a genome browser such as, for example, the genome browser of UCSC (http://genome-euro.ucsc.edu/), which provides a graphic scheme of the genomic location including additional information on the encoded sequences etc., can be used to derive molecular and positioning information on each DMP loci identifier as depicted in Table 1 and 1a. For instance, upon introducing the DMP's Illumina ID as shown the Tables (cg...) into the genome browser coordinates of the genomic location of the DMP can be retrieved. Coordinates of genomic location of the DMP may accordingly be used for the design and synthesis of adjacent or binding polynucleotides, e.g., primer sequences, or for sequence analysis purposes etc. The Illumina ID nomenclature is constructed on CG loci designation which is based on actual or contextual sequences of each CpG locus and takes advantage of the sequences flanking the DMP. Thereby the ID is unaffected by reference genome changes or differences in genome versions. This allows for an unambiguous assignment of the ID to genomic coordinates.

Further, the Illumina Infinium HumanMethylation450 BeadChip array system (or any future and functionally equivalent variant thereof) may be used to further analyze or assess the DMP shown in Tables 1 or 1a.

The term "TNM-classification" as used herein relates to a system for classifying a malignancy, which is typically used in solid tumors and allows for or assists in prognostic cancer staging. The system is primarily based on the assessment of tumor (T), nodes (N) and distant metastasis (M).

The T-descriptor of the TNM classification is used to describe the size of the primary tumor and its invasion into adjacent tissues. For example, T0 indicates that no evidence of tumor is present, while T1-T4 are used to identify the size and extension of the tumor, with progressive enlargement and invasiveness from T1 to T4. T-values are typically assessed differently based on the involved anatomic structures.

The N-descriptor of the TNM classification is used to describe regional lymph node involvement of the tumor since lymph nodes function as biological filters. N0 typically indicates no regional nodal spread, while N1-N3 indicates some degree of nodal spread, with a progressively distal spread from N1 to N3.

The M-descriptor of the TNM classification is used to identify the presence of distant metastases of the primary tumor. Metastasis is given when the tumor spreads beyond regional lymph nodes. A tumor is classified as M0 if no distant metastasis is present and M1 if there is evidence of distant metastasis.

An overview of the TNM classification of lung cancer as exemplarily used in the context of the present invention may be derived from suitable sources such as the 8^{th} edition of the TNM staging for lung cancer or from the following Table 2, which depicts TNM classification for lunger cancer. Similar classification schemes for other cancer types would be known to the skilled person.

**Table 2**

| **Primary tumor (T)** | |
|---|---|
| Tx | Tumor that is proven histopathologically (malignant cells in bronchopulmonary secretions/washings) but cannot be assessed or is not demonstrable radiologically or bronchoscopically |
| T0 | No evidence of primary tumor |
| Tis | Carcinoma in site |
| T1 | Size ≤ 3 cm Airway location: In or distal to the lobular bronchus Local invasion: None (surrounded by lung or visceral pleura) |
| T1mi | Minimally invasive adenocarcinoma (pure lepidic pattern, ≤ 3 cm in greatest dimension and ≤ 5 mm invasion |
| T1a | Size ≤ 1 cm |
| T1b | Size >1 cm but ≤ 2 cm |
| T1c | Size > 2 cm but ≤ 3 cm |
| T2 | Any of the following characteristics: Size > 3 cm but ≤ 5 cm Airway location: Invasion of the main bronchus (regardless the distance to the carina) or presence of atelectasis or obstructive Pneumonitis that extends to hilar region (whether it is involving part or the entire lung) Local invasion: visceral pleura (PL1 or PL2) |
| T2a | Size > 3 cm but ≤ 4 cm |
| T2b | Size > 4 cm but ≤ 5 cm |
| T3 | Any of the following characteristics Size > 5 cm but ≤ 7 cm Local invasion: direct invasion of chest wall (including superior sulcus tumors), parietal pleura (PL3), phrenic nerve or parietal pericardium Separate tumor nodule(s) in the same lobe of the primary tumor |
| T4 | Any of the following characteristics Size > 7 cm Airway location: Invasion of the carina or trachea Local invasion: Diaphragm, mediastinum, heart, great vessels, recurrent laryngeal nerve, esophagus or vertebral body Separate tumor nodule(s) in an ipsilateral different lobe of the primary tumor |

| **Lymph nodes (N)** | |
|---|---|
| Nx | Regional lymph nodes cannot be evaluated |
| N0 | No regional lymph nodes involvement |
| N1 | Involvement of ipsilateral peribronchial and/or ipsilateral hilar lymph nodes (includes direct extension to intrapulmonary nodes) |
| N2 | Involvement of ipsilateral mediastinal and/or subcarinal lymph nodes |
| N3 | Involvement of any of the following lymph node groups: contralateral mediastinal, contralateral hilar, ipsilateral of contralateral scalene or supraclavicular nodes |

| **Distant metastasis (M)** | |
|---|---|
| M0 | No distant metastasis |
| M1 | Presence of distant metastasis |
| M1a | Separate tumor nodule(s) in a contralateral lobe to that of the primary tumor or tumors with pleural or pericardial nodules or malignant effusion |
| M1b | Single extrathoracic metastasis |
| M1c | Multiple extrathoracic metastases to one or more organs |

According to the present invention the diagnosing, detecting or monitoring of a cancer disease in a subject comprises a distinction between the T-descriptor T1 and T3/T4 in the subject, preferably the T-descriptor T1 and T3/T4 in a subject having lunger cancer or being suspected of having lung cancer. For example, a distinction may be made between a subject which typically shows a tumor size of ≤ 3 cm with an airway location in or distal to the lobular bronchus and no local invasion, which corresponds to status T1, and a subject which typically shows any of a tumor size of > 5 cm but ≤ 7 cm, a local invasion in the form of a direct invasion of chest wall (including superior sulcus tumors), parietal pleura (PL3), phrenic nerve or parietal pericardium, and separate tumor nodule(s) in the same lobe of the primary tumor, which corresponds to status T3, and/or a subject which typically shows any of a tumor size of > 7 cm, an invasion of the carina or trachea, a local invasion of diaphragm, mediastinum, heart, great vessels, recurrent laryngeal nerve, esophagus or vertebral body and the presence of separate tumor nodule(s) in an ipsilateral different lobe of the primary tumor, which corresponds to status T4.

The method according to the present invention, based on the determination of the methylation level of the methylation site comprising the DMP cg01218619 (SEQ ID NO: 1), thus allows to determine whether a subject is affected by a tumor of TNM-classification T-descriptor T1 or T3/T4. In preferred embodiments, the method according to the present invention, based on the determination of the methylation level of the methylation site comprising the DMP cg01218619 (SEQ ID NO: 1), allows to determine whether a subject is affected by a lung tumor of TNM-classification T-descriptor T1 or T3/T4.

A correlation of the above mentioned methylation site with a TNM-classification within the context of the present invention may thus be based on the presence of a differentially methylated position (DMP), which shows a methylation status variation in a genomic DNA derived from a subject's sample if said subject belongs to a T1 TNM-classification group as defined herein above in comparison to a subject belonging to an T3/T4 TNM-classification group as defined herein above. The differential methylation pattern underlying the present invention and enshrined in the DMP of Table 1 is not restricted to a simple pattern of non-methylated in the one group and methylated in the other group, or vice versa, but reflects combinatorial methylation situations (methylated or unmethylated) in all samples derived form a subject.

In preferred embodiments, the method according to the present invention comprises performing a statistical prediction of the affiliation of a subject to a TNM-classification group as defined herein. The statistic prediction may comprise any suitable approach or algorithm known to the skilled person. In particularly preferred embodiments, the statistical prediction utilizes a machine learning approach trained on an appropriately representative training cohort. "Machine learning" as used herein typically relies on a two-step approach: first, a training phase; and second, a prediction phase. In the training phase, values of one or more parameters of the machine-learning model (MLM) are set using training techniques and training data. In the prediction phase, the trained MLM operates on measurement data. Building an MLM can include the training phase to determine the values of the parameters. Building an MLM can generally also include determining values of one or more hyperparameters. Typically, the values of one or more hyperparameters of the MLM are set and not altered during the training phase. Hence, the value of the hyperparameter can be altered in outer-loop iterations; while the value of the parameter of the MLM can be altered in inner-loop iterations. Sometimes, there can be multiple training phases, so that multiple values of the one or more hyperparameters can be tested or even optimized. The performance and accuracy of most MLMs are strongly dependent on the values of the hyperparameters. Example hyperparameters include: number of layers in a convolutional neural network; kernel size of a classifier kernel; input neurons of an ANN; output neurons of an ANN; number of neurons per layer; learning rate; etc. Various types and kinds of MLMs can be employed in the context of the present invention. For example, a novelty detector MLM / anomaly detector MLM, or a classifier MLM may be employed, e.g., a binary classifier. For example, a deep-learning (DL) MLM can be employed: here, features detected by the DL MLM may not be predefined, but rather may be set by the values of respective parameters of the model that can be learned during. As a general rule, various techniques can be employed for building the MLM. For example, typically, the type of the training can vary with the type of the MLM. Since the type of the MLMs can vary in different implementations, likewise, the type of the employed training can vary in different implementations. For example, an iterative optimization could be used that uses an optimization function that is defined with respect to one or more error signals. For example, a backpropagation algorithm can be employed.

The term "representative training cohort" as used in the context of the machine learning algorithms described above refers to a data set which has been successfully associated with TNM-classification groups as defined herein. In particular, the term relates to a data set in which all parameters (including the target parameter to be predicted) are known and curated. Importantly, the ratio of cases per group is representative of the objective of the model to be trained.

A suitable example of a machine learning approach which may be used in the context of the statistical prediction according to the present invention is logistic regression. "Logistic regression" is a supervised machine learning algorithm which aims at a binary classification by predicting the probability of an event or observation. It typically analyzes the relationship between two or more independent variables and classifies the data subsequently into discrete classes. As such it may be used as part of predictive modeling for estimating the mathematical probability of an observation to belong a specific category or not. Further information would be known to the skilled person or can be derived from suitable literature references or textbooks such as Maalouf, 2011, International Journal of Data Analysis Techniques and Strategies, 3, 3, 281-299.

A further suitable example of a machine learning approach which may be used in the context of the statistical prediction according to the present invention is the Random Forest algorithm. "Random Forest" is a machine learning algorithm which combines the output of multiple decision trees to reach a single result. Decision trees typically start with a basic question which develops into more complex questions concerning a certain subject. These questions build decision nodes in a tree, acting as a means to split the data. Each question helps to arrive at a final decision. Decision trees typically seek to find the best split to subset the data, and are trained through, e.g. the Classification and Regression Tree (CART) algorithm. Metrics, such as Gini impurity, information gain, or mean square error (MSE), may be used to evaluate the quality of the split. Based on decision tree classifiers ensemble learning methods are used to aggregate the results of the classifiers. The Random Forest approach accordingly creates an uncorrelated forest of decision trees and selects a subset of available features in order to arrive at certain prediction.

According to the present invention the DNA molecule, i.e. genomic DNA molecule, for subsequent analysis may be derived from a subject's sample. The sample may be any suitable sample type or form. The sample may, for example, have been obtained from a subject. In preferred embodiments, the sample is a blood, e.g. plasma or serum, urine, feces, saliva, or liquid biopsy sample. In other embodiments the sample may be a tissue, e.g., tissue biopsy, semen, liquor, or other sample. The present invention preferably envisages the use of liquid biopsy samples derived from blood/urine or other body fluids. Also envisaged is to make use of previously deposited samples. In certain embodiments the sample is a cell free DNA (cfDNA) sample. The term "cfDNA" as used herein refers to degraded DNA fragments released to body fluids such as blood plasma, urine, cerebrospinal fluid, etc., wherein the cfDNA typically has a short half-life and requires rapid processing and/or stabilization. The typical size of cfDNA fragments is about 160 bp. cfDNA comprises various forms of DNA freely circulating in body fluids such as circulating tumor DNA (ctDNA) or cell-free mitochondrial DNA (cf mtDNA). In specific embodiments the sample may also be a genomic DNA molecule sample, which can be derived, e.g., from a tissue obtained in a biopsy. It is particularly preferred that the sample is blood plasma sample.

In certain embodiments, the sample may have been obtained, for comparison reasons, from a healthy subject or a cancer patient, e.g. a subject having an established T1 or T3/4 status, or a cohort of such subjects or patients as described herein above, i.e., a patient or subject whose disease status has been clarified before or independently. The sample may further have been derived from any patient or subject afflicted by a cancer disease or suspected to be afflicted by a cancer disease, or from any other subject, e.g., for control or reference purposes or from any subject independent of suspicion of being afflicted by a cancer disease, e.g., for a cancer screening. In certain embodiments it is also envisaged to make use of previously deposited samples.

The term "subject" as used herein refers to any individual who is to be examined for cancer, preferably to any individual whose affiliation to a TNM-classification group as defined herein shall be determined. The subject may, for example, be a patient which is suspected to be affected by a cancer disease, e.g. by lung cancer. The subject may be human, female or male (or diverse). The gender has no influence on the outcome of the cancer detection in the context of the TNM classification according to the present invention.

In a further embodiment, the diagnostic and/or monitoring conclusion with respect to the presence of a cancer disease in the subject, i.e. with respect to a TNM classification as defined herein above, may additionally be based on the evaluation of further data. Such further data may, for example, be the subject's imaging-based data. Examples of contemplated imaging-based data are ultra-low-dose CT screening data, MRT data, medical images and/or pathology image. These data, which may themselves already be connected to or translated into a diagnostic output for a subject, may be combined with the conclusions derived from the method of diagnosing, detecting or monitoring a cancer disease as described herein above. The results of the evaluation(s) contribute to the diagnostic and/or monitoring conclusion with respect to the presence of a cancer disease in the subject as defined herein, in particular the distinction between a TNM-classification T1 status and T3/T4 status in the subject.

In the alternative or in addition, i.e., in a further preferred embodiment the diagnostic or monitoring conclusion with respect to the presence of a cancer disease in the subject, in particular with respect to a TNM classification as defined herein, may additionally be founded on non-imaging-based data, i.e. such non-imaging data may suitably be evaluated. Examples of contemplated non-imaging-based data are data on age, sex, race, characteristics of cancer phenotype, histologic characteristics, histologic subtype, detectable molecular changes, preferably on the level of the genome, transcriptome, metabolome, proteome, glycome or lipidome, biomarkers and/or laboratory tests. These data may be combined with the conclusions derived from the method of diagnosing, detecting or monitoring a cancer disease as described herein above and optionally with the conclusions including the imaging-based data. The results of the evaluation(s) accordingly contribute to the diagnostic and/or monitoring conclusion with respect to the presence of a cancer disease in the subject, in particular with respect to a TNM classification as defined herein. For example, the data may be combined with conclusions and other data entities via multimodal data integration. In specific embodiments, the combination input data may, based on suitable weighing algorithms, result in a further, more complete overall diagnostic and/or monitoring conclusion.

In a further preferred embodiment of the present invention, the method for diagnosing, detecting or monitoring a cancer disease in a subject comprises the determination of the methylation level of the methylation site by modifying the genomic DNA molecules to allow for determination of the methylation status of methylation sites, preferably by bisulfite-conversion or enzymatic conversion, and determining the sequence of the genomic DNA molecules, thereby also determining the methylation status of the genomic DNA molecule.

The "modification of DNA molecules" may be based on any technology which allows for the determination of the methylation status of methylation sites. It is preferred that the modification is a bisulfite-conversion or enzymatic conversion as described in detail herein. The method typically comprises a contacting of correspondingly modified DNA molecules with complementary polynucleotides resulting in a binding or hybridization reaction with complementary sequences.

As a subsequent step the method comprises a step of determining the sequence of the molecules. The sequence data of the previously modified DNA molecules may be obtained with any suitable technology, preferably in a high-throughput approach. This typically includes next-generation sequencing (NGS) or second-generation sequencing techniques. Such approaches comprise any sequencing method that determines the nucleotide sequence of either individual nucleic acid molecules or expanded clones for individual nucleic acid molecules in a highly parallel fashion. The sequencing may be performed according to any suitable massive parallel approach. In certain embodiments, the sequencing may include a previous preparation of nucleic acids, the sequencing, as well as subsequent imaging and initial data analysis steps. Preparation steps may, for example, include amplification steps, e.g., PCR amplification, randomly breaking nucleic acids into smaller sizes and generating sequencing templates such as fragment templates. Spatially separated templates can, for example, be attached or immobilized on solid surfaces which allows for multiple sequencing reactions to be performed simultaneously. In typical examples, a library of nucleic acid fragments may be generated and adaptors containing universal priming sites may be ligated to the end of the fragments. Subsequently, the fragments can be denatured into single strands and captured by beads.

Correspondingly obtained data are provided in the form of sequencing reads which may be single-end or paired-end reads. Obtaining such sequencing data may further include the addition of assessment steps or data analysis steps.

Furthermore, the presently described methodology may be used with any suitable sequencing read length. It is preferred to make use of sequencing reads of a length of about 50 to about 170, e.g., 50, 60, 70, 80, 90, 100, 110, 120, 130, 140, 150, 160, 170 or more nucleotides or any value in between the mentioned values. Particularly preferred is paired-end sequencing with 2 x 75 bp reads or with 2 x 150 bp reads.

The elucidation of the sequence of the DNA molecules allows to determine the methylation status of the molecules. For example, as described above, in the DNA sequencing, uracil, which is turned to thymine during amplification, is recognized as thymine during sequencing and on the opposite strand adenine is provided due to base pairing, while methylated cytidines are paired with guanines. C->U transitions at the unmethylated sites can accordingly be detected, e.g., by comparing the sequences with reference sequences or, for example, parallel sequencing results with non-bisulfite converted or non-enzymatic converted DNA molecules. Accordingly, in certain embodiments, the present invention envisages a double or parallel approach wherein DNA molecules from a sample (i) are modified by the bisulfite or enzymatic conversion; and (e.g., a portion of the sample) (ii) are not modified. Both groups of molecules are subsequently analyzed as described above, e.g., by determining the sequence. In further embodiments Methylation-Specific MLPA (multiplex ligation-dependent probe amplification) may be used. Finally, a comparison of both sequences may be performed to detect methylated positions. This may further be compared with sequences stored in databases comprising information on said DMP or CpG-site. Examples of suitable databases include, for example, KEGG (Kanehisa and Goto, 2000, Nucleic Acids Research, 28, 1, 27-30) or GO (Gene Ontology Consortium, 2004, Nucleic Acids Research, 32, Suppl 1, D258-D261).

In a further embodiment, the method comprises the reporting of the diagnostic conclusion, i.e. the TNM-classification of the subject. A corresponding report may be provided in any suitable manner or form, e.g. as electronic file, as electronic file distributed or accessible over the internet, e.g. provided in a cloud or deposited on a server, or web-based, e.g. provided on a suitable website. Alternatively, the report may be provided in paper form. The report may be provided to a subject or another person associated with the subject, a scientist or group of scientists, a judicial authority, a hospital or clinic, a medical practitioner, or a government office (including information relevant for these entities). The report may accordingly be redacted, modified, extended or adjusted to the above specified recipient.

In a further aspect, the present invention relates to a kit comprising, or alternatively consisting of, probes, preferably polynucleotide probes, for detecting the DMP corresponding to Illumina probe ID cg01218619 (SEQ ID NO: 1). The probes may have any suitable length. Preferably, they comprise 50 to 120 nucleotides, e.g., 50, 55, 60, 65, 70, 75, 80, 85, 90, 95, 100, 105, 110, 115, 120, 124, 130, 135 or 140, nucleotides, or any value in between the mentioned values. In particularly preferred embodiments, they have a length of about 80 or about 120 nucleotides. For example, the probe may be designed to hybridize to a DMP as shown in Table 1, e.g. according to conditions mentioned herein above. In a particularly preferred embodiment, the DMP of Table 1 may be represented by a number of 1 set of probes, e.g. probes having the sequences of SEQ ID NO: 1 or reverse complements thereof. Also envisaged is a higher number of probes, e.g., if a different target coverage is intended, or if additional DMPs not mentioned in Table 1 are considered to be of interest, the kit according to the present invention may comprise one or more additional probes which may be further included. Such additional probes may, for example, be identified in further future analyses or be derived from known cancer, e.g. lunger cancer, patients.

The probes of the kit according to the present invention may, in certain embodiments, be linked or tethered to a solid support such as an array structure or the like. Also, a link, e.g., via biotin-streptavidin interaction, with magnetic beads and a corresponding actuation and fixation by magnetic forces is envisaged. Alternatively, the probes may be free floating in a liquid medium.

In a further aspect, the present invention relates to a method of enriching DNA molecules derived from a subject's sample, e.g., as described above comprising: (a) modifying the DNA molecules to allow for determination of the methylation status of methylation sites; (b) contacting the modified molecules, i.e. the bisulfite-converted or enzymatically converted molecules, with at least one polynucleotide probe selected from the polynucleotide probes as described herein and enriching modified DNA molecules by hybridization capture.

The modification of DNA molecules and the contacting of correspondingly modified DNA molecules with polynucleotides has been described herein above. The binding is a sequence-specific hybridization which allows to specifically collect only DNA molecules which show a high degree of complementarity with the polynucleotide probes, e.g., 95%, 96%, 97%, 98%, 99%, or 100%. The binding specificity may be adjusted by changes to the hybridization conditions, e.g., temperature, pH, ionic concentrations etc. The polynucleotide probes may have any suitable length, preferably a length of 50-150 nucleotides, more preferably of 120 nucleotides. The polynucleotide probes may be free floating or be tethered or linked to a support, e.g., as described herein in detail, to allow for pulling down binding DNA molecules. Also, the alternative use of magnetic beads and a corresponding magnetic actuation approach is envisaged. Further contemplated are antibody-based techniques, as well as molecular approaches based on biotin/streptavidin interactions.

Subsequent to the contacting step, the bound DNA molecules are "enriched", i.e., only DNA molecules which are in fact specifically bound to the polynucleotide probes are kept or retained, whereas other, non-bound or not specifically bound DNA molecules or other components in a mixture are discarded. The enriched DNA molecules may be used for further analysis steps or stored for future activities. Enriched DNA molecules may, in additional embodiments, further be amplified. Also contemplated is a spiking with specific indices that allow for a sequencing of the molecules.

As a further step, which may be optional, the method comprises a step of determining the sequence of the enriched molecule as described herein above.

In a further aspect, the present invention also envisages the use of a DMP defined herein, preferably of a DMP corresponding to Illumina probe IDs cg01218619 (SEQ ID NO: 1) for diagnosing, detecting or monitoring a cancer disease in a subject wherein the diagnosing, detecting or monitoring of a cancer disease comprises a distinction between a TNM-classification T1 status and T3/T4 status in the subject as described herein. In additional embodiments, the present invention also envisages the use of a kit as defined herein above, for the binding and/or analysis of a DMP as defined in Table 1. The kit is preferably used for diagnosing, detecting or monitoring a cancer disease as describe herein wherein the diagnosing, detecting or monitoring of a cancer disease in a subject comprises a distinction between a TNM-classification T1 status and T3/T4 status in the subject.

The following figures and examples are provided for illustrative purposes. It is thus understood that the figures and examples are not to be construed as limiting. The skilled person in the art will clearly be able to envisage further modifications of the principles laid out herein.

### EXAMPLES

### 1. METHOD

Methylation is an epigenetic modification of the DNA, acting as a repressive mark that thereby regulates transcription of genes. DNA methylation can be detected and assessed using a variety of technological methods including mass spectrometry, methylation-specific PCR (MSP), pyrosequencing and next generation sequencing (NGS)-based technologies (Kurdyukov & Bullock, 2016, DNA Methylation Analysis: Choosing the Right Method. Biology (Basel), 5(1)). DNA subjected for analyses can either be genomic DNA (requires fragmentation into smaller fragments) or cell-free DNA isolated from liquid biopsies (naturally occurring in the size of about 160 base pairs). Prior to the enrichment step, the DNA needs to be modified to prepare for later NGS sequencing (library preparation) and to discriminate methylated and unmethylated cytosine in the CpG sites (conversion of unmethylated cytosine residues). Since common amplification (e.g., PCR) and sequencing methods (e.g., pyrosequencing and NGS) cannot distinguish between unmethylated cytosine (C) and methylated cytosine (i.e., 5-methylcytosine [C-m] and 5-hydroxymethylcytosine [C-hm]), a conversion of the unmethylated cytosines is necessary as a pre-step. This conversion can be conducted via a biochemical or an enzymatic reaction allowing adequate discrimination between methylated and unmethylated cytosines. Both methods convert unmethylated cytosines (C) to uracils (U). Subsequent PCR amplification turns the uracils to thymidins (T), whereas methylated cytosines remain unconverted. This conversion step allows for discrimination of methylated and unmethylated cytosine.

The capture of the CpG sites of interest can be performed using biotinylated probes/baits (nucleic acids RNA or DNA) able to facilitate hybridization with the surrounding sequences of the CpG of both strands. To fully capture the entire complexity of the CpG site of interest, a combination of baits, targeting the leading and the lagging strand, as well as targeting different conversion states (methylated and unmethylated after conversion) is required.

Since the number of CpG sites in the whole genome is about 28 million (Lovkvist et al., 2016, Nucleic Acids Res. 2016; 44(11) :5123-32), the samples must be enriched for the regions of interest to reduce the number of analyzed CpG sites and thus allow streamlined and cost-efficient analysis. After the hybridization, the enrichment of the regions of interest can be performed by magnetic beads that bind biotinylated fragments hybridized to the fragments containing the sites of interest.

After the conversion and amplification, subsequent analyses can distinguish methylated (remained Cs) and unmethylated (converted to Ts) cytosines. NGS approaches include targeted methods such as amplicon methyl-seq or target enrichment, as well as whole-genome sequencing. To assess the sequencing results, a bioinformatical procession is performed that aligns the resulting reads to appropriate reference genomes and analyzes them regarding their DNA methylation status.

### 2. EXPERIMENT

DNA methylation patterns were assessed on cell-free derived DNA (cfDNA) extracted from blood plasma of lung cancer patients. cfDNA extraction was performed according to manufacturer's instructions (Qiagen - QIAamp MinElute ccfDNA Midi Kit) . An enzymatic reaction for DNA methylation conversion was followed by NGS-based hybrid capture target enrichment using a personalized enrichment panel designed by reagent provider Twist. The regions of interest were enriched by hybridization to specifically designed bait probes. In total, the reagent panel contained four probes against the methylated and unmethylated state for both strands of the differentially methylated position (DMP) corresponding to Illumina probe ID cg01218619 (SEQ ID NO: 1), thus allowing enrichment of the specific DMP.

NGS sequencing was performed with 25 mrp using 300-cycle high output cartridges and 25 ng cfDNA input. The raw sequencing data achieving a conversion rate >99.5% were analyzed using a bioinformatic pipeline.

## Claims

1. A method for diagnosing, detecting or monitoring a cancer disease in a subject, comprising determining the methylation level of a methylation site in a genomic DNA molecule derived from the subject's sample, wherein said methylation site comprises the differentially methylated position (DMP) corresponding to Illumina probe ID cg01218619 (SEQ ID NO: 1) and wherein the diagnosing, detecting or monitoring of a cancer disease in a subject comprises a distinction between a TNM-classification T1 status and T3/T4 status in the subject.

2. The method of claim 1, wherein the diagnosing, detecting or monitoring of a cancer disease in a subject comprises performing a statistical prediction.

3. The method of claim 2, wherein said statistical prediction utilizes a machine learning approach such as logistic regression or random forest classification trained on an appropriately representative training cohort.

4. The method of any one of claims 1 to 3, additionally comprising the step of evaluation of
(i) the subject's imaging-based data, ultra-low-dose CT screening, medical images and/or pathology images; and/or
(ii) the subject's non-imaging-based data, such as data on age, sex, race, characteristics of cancer phenotype, histologic characteristics, stage of development of cancer, histologic subtype, detectable molecular changes, preferably on the level of the genome, transcriptome, metabolome, proteome, glycome or lipidome, biomarkers and/or laboratory tests.

5. The method of claim 4, wherein the results of the evaluation(s) contribute to the diagnostic conclusion with respect to the presence of a cancer disease in the subject, preferably with respect to a distinction between a TNM-classification T1 status and T3/T4 status in the subject.

6. The method of any one of claims 1 to 5, wherein the determination of the methylation level of the methylation site comprises modifying the genomic DNA molecules to allow for determination of the methylation status of methylation sites, preferably by bisulfite-conversion or enzymatic conversion, and determining the sequence of the genomic DNA molecules, thereby also determining the methylation status of the genomic DNA molecule.

7. The method of any one of claims 1 to 6, comprising the step of reporting the diagnostic conclusion, preferably the TNM-classification of the subject, more preferably in an electronic form.

8. A kit comprising probes for detecting the differentially methylated position (DMP) corresponding to Illumina probe ID cg01218619 (SEQ ID NO: 1).

9. A method of enriching DNA molecules derived from a subject's sample comprising:
(a) modifying the DNA molecules to allow for determination of the methylation level of methylation sites, preferably by bisulfite-conversion or enzymatic conversion; and
(b) contacting the modified molecules with at least one probe as defined in claim 8 and enriching the modified DNA molecules by hybridization capture.

10. The method of claim 9, additionally comprising the step (c) of determining the sequence of the enriched molecules, thereby also determining the methylation level of the enriched molecules.

11. The method of claim 10, wherein the determined methylation level is statistically evaluated to allow for diagnosing, detecting or monitoring a cancer disease in the subject, wherein the diagnosing, detecting or monitoring of a cancer disease in the subject comprises a distinction between a TNM-classification T1 status and T3/T4 status in the subject.

12. The method of any one of claims 1 to 7 or 9 to 11, wherein said sample is a blood, plasma, urine, feces, saliva, or liquid biopsy sample.

13. Use of a differentially methylated position (DMP) corresponding to Illumina probe ID cg01218619 (SEQ ID NO: 1) for diagnosing, detecting or monitoring a cancer disease in a subject, wherein the diagnosing, detecting or monitoring of a cancer disease comprises a distinction between a TNM-classification T1 status and T3/T4 status in the subject.

14. The method of any one of claims 1 to 7, or 11 to 12, or the use of claim 13, wherein said cancer disease is lung cancer.
